# EUROPÄISCHE PATENTSCHRIFT

(11) **EP 2 116 190 B1**
(45) Veröffentlichungstag und Bekanntmachung des Hinweises auf die Patenterteilung: **23.09.2015**
(21) Anmeldenummer: 09005202.8
(22) Anmeldetag: 09.04.2009
(51) Int. Cl.: A61B 17/00, A61B 17/30

(54) **Verschlusselement für ungewollte Öffnungen im Herzen**
Closing element for unwanted openings in the heart
Elément de fermeture pour ouvertures non souhaitées dans le coeur

(30) Priorität: 07.05.2008 DE 102008022673
(43) Veröffentlichungstag der Anmeldung: 11.11.2009
(73) Patentinhaber: Peter Osypka Stiftung Stiftung des bürgerlichen Rechts, 79639 Grenzach-Wyhlen (DE)
(72) Erfinder: Osypka, Peter, 79618 Rheinfelden-Herten (DE)
(74) Vertreter: Kunst, Manuel Nikolaus Johannes

(56) Entgegenhaltungen:
- WO-A2-2008/036384
- US-A1- 2003 055 455
- US-A1- 2003 225 402
- US-A1- 2008 249 562

## Beschreibung

Die Erfindung betrifft ein Verschlusselement für ungewollte Öffnungen im Herzen für Vorhofseptum-Defekte (ASD), welche aus Fäden aus körperverträglichem Metall und/oder Kunststoff, aus absorbierbarem Kunststoff, aus Memory-Materialfäden, aus Memory-Kunststoff-Fäden, aus Nitinoldrähten oder aus mit Kunststoff ummantelten Drähten oder Fäden gebildet oder geflochten ist, wobei dieses Verschlusselement zwei aus den Fäden gebildete Verschlussscheiben und zwischen diesen einen die Öffnung im Herzen in Gebrauchsstellung durchsetzenden Bereich geringerer radialer Abmessung aufweist und die Verschluss-Scheiben die Ränder der ungewollten Öffnung im Herzen in Gebrauchsstellung beidseits übergreifen, wobei beide Verschlussscheiben jeweils eine die sie bildenden Fäden an einer Seite festlegende, von der Verschlussscheibe in radialer Richtung überragte Fassung aufweisen, diese Fassungen zur Bildung eines Abstands zwischen den beiden Verschlussscheiben und zur Bildung des Bereichs mit der verminderten radialen Abmessung in Gebrauchsstellung miteinander verbunden sind, die Fassung der einen Verschlussscheibe eine axiale, nach außen reichende Öffnung oder Ausnehmung hat, in welche ein Vorsprung der Fassung der zweiten Verschlussscheibe einführbar und in dieser Lage damit verbindbar ist und wobei der Vorsprung der einen Fassung in die Öffnung oder Ausnehmung oder Vertiefung der anderen Fassung formschlüssig passt, damit verschraubbar und/oder verschweißbar ist.

Ein derartiges Verschlusselement ist aus der US2003/0055455 A1 bekannt. Die dabei vorgesehenen Fassungen und Kupplungen der beiden Verschlussscheiben machen jedoch zwei Einführinstrumente, nämlich für jede Verschlussscheibe ein Einführinstrument, erforderlich. Die Verbindung der beiden Verschlussscheiben kann somit erst im Herzen erfolgen, was einen zusätzlichen operativen Aufwand bedeutet.

Es besteht deshalb die Aufgabe, ein Verschlusselement der eingangs genannten Art zu schaffen, dessen Verschlussscheiben schon verbunden mit einem Zuführelement erfasst und an den Ort seiner Bestimmung gebracht werden kann.

Zur Lösung dieser Aufgabe ist das eingangs definierte Verschlusselement dadurch gekennzeichnet, dass eine einzige Fassung eine von der Verbindungsstelle mit der anderen Fassung abgewandte Kupplungsstelle oder Öffnung für den lösbaren Angriff eines Einführinstruments aufweist und dass die Kupplungsstelle oder Öffnung ein Gewinde hat, zu dem ein Gegengewinde des Einführinstruments passt, dass die Fassung einer einzigen Verschlussscheibe eine von der Verbindungsstelle mit der Fassung der anderen Verschlussscheibe abgewandte Kupplungsstelle oder Öffnung zum Verbinden mit dem Zuführinstrument aufweist, wobei die Fäden oder die Maschenweite des Geflechts der Verschlussscheibe einen Durchtritt für das Zuführinstrument freigibt oder freilässt, und dass die Kupplungsstelle oder Öffnung für das Einführinstrument an derjenigen Fassung vorgesehen ist, welche den Vorsprung zum Kuppeln mit der Fassung der anderen Verschlussscheibe trägt. Das Verschlusselement kann also aus zwei zunächst einzelnen Verschlussscheiben gebildet werden, die an den Fassungen verbunden werden, womit das Geflecht beziehungsweise die Fäden dieser Verschlussscheiben zusammengehalten sind, so dass die Fassungen zwischen den beiden Verschlussscheiben zu liegen kommen, also an keiner Verschlussscheibe eine derartige Fassung nach außen überstehen muss, die den Blutfluss in den Herzvorhöfen negativ beeinflussen könnte. Dabei ergibt sich nicht nur eine zugfeste, sondern im Falle einer Verschweißung sogar eine nicht mehr lösbare Verbindung der beiden Verschlussscheiben mit entsprechend großer Stabilität.

Ferner ist eine lösbare Verbindung des Einführinstruments mit dem Verschlusselement auf einfache Weise möglich. Beispielsweise kann die Öffnung an der Fassung ein Innengewinde und das Einführinstrument an seinem distalen Ende ein dazu passendes Außengewinde haben.

Dadurch, dass die Fassung einer Verschlussscheibe eine von der Verbindungsstelle mit der Fassung der anderen Verschlussscheibe abgewandte Kupplungsstelle oder Öffnung zum Verbinden mit dem Zuführinstrument aufweist, wobei die Fäden oder die Maschenweite des Geflechts der Verschlussscheibe einen Durchtritt für das Zuführinstrument freigibt oder freilässt, kann das eine Zuführinstrument auf einfache Weise durch das in der Regel weitmaschige und somit nachgiebige Geflecht einer solchen Verschlussscheibe hindurchgeschoben werden, um eine der zwischen den Verschlussscheiben befindlichen Fassungen und dabei diejenige Fassung zu erreichen, die die Kupplungsstelle zum Verbinden mit diesem Zuführinstrument aufweist. Somit kann das Verschlusselement in an sich bekannter Weise mit einem Zuführelement erfasst und an den Ort seiner Bestimmung gebracht werden, ohne dass eine Fassung oder Hülse auf einer Seite des Verschlusselements außen überstehen muss und gegebenenfalls den Blutfluss in einem der Vorhöfe beeinträchtigen könnte. Besonders günstig ist es, wenn die in Gebrauchsstellung einander zugewandten Fassungen der beiden Verschlussscheiben zugfest miteinander verbunden sind. Somit können sie einerseits den Vorgang der Implantation und andererseits die bei Gebrauch eventuell auftretenden Kräfte gut aufnehmen, ohne auseinander bewegt werden zu können.

Weil die Kupplungsstelle oder Öffnung für das Einführinstrument an derjenigen Fassung vorgesehen sein kann, welche den Vorsprung zum Kuppeln mit der Fassung der anderen Verschlussscheibe trägt, hat diese Fassung genügend Material und Substanz, um eine Öffnung darin anbringen zu können, während die andere Fassung ihrerseits schon eine Aufnehmung oder Vertiefung hat, so dass daran eine weitere Vertiefung für das Einführinstrument nur durch eine entsprechende Verlängerung möglich wäre.

Eine vorteilhafte Ausgestaltung kann vorsehen, dass die beiden Scheiben in Gebrauchsstellung federnd miteinander verbunden sind, also der Abstand gegen eine Federkraft etwas geändert werden kann, um eine feste Anlage der beiden Scheiben beidseits einer Herzwand zu erlauben und dabei unterschiedlich dicke Herzwände berücksichtigen zu können.

Die axial verlaufende Öffnung oder Ausnehmung der Fassung der einen Verschlussscheibe kann ein Innengewinde und der dazu passende Vorsprung der Fassung der zweiten Verschlussscheibe ein dazu passendes Gewindestück oder Außengewinde für eine gegenseitige Verschraubung der beiden Fassungen aufweisen. Somit kann durch eine derartige Verschraubung die zugfeste Verbindung hergestellt und durch eine zusätzliche Verschweißung noch gesichert werden.

Die Außenkontur wenigstens einer der Verschlussscheiben kann kreisrund oder von einem Kreis abweichend geformt sein. Die Verschlusselemente können also auch an unterschiedliche anatomische Verhältnisse angepasste Umfangskonturen haben.

Eine einfache Ausführungsform ergibt sich, wenn die beiden Verschlussscheiben gleich groß oder insbesondere die im rechten Vorhof anzuordnende Verschlussscheibe kleiner als die andere Verschlussscheibe ist. Vor allem die genannte Alternative erlaubt es, die unterschiedlichen Blutdrücke in den beiden Vorhöfen zu berücksichtigen und die größere Verschlussscheibe in dem linken Vorhof anzuordnen, in welchem sie einem höheren Blutdruck standhalten soll.

Eine abgewandelte Ausführungsform kann vorsehen, dass die Verschlussscheibe für den rechten Vorhof eine geringe Anzahl von Fäden oder Drähten als die Verschlussscheibe für den linken Vorhof aufweist. Dies ist möglich, weil der Blutdruck im rechten Vorhof geringer als im linken Vorhof ist. Somit kann die Zahl der Drähte oder Fäden für den rechten Vorhof reduziert sein, dass heißt es ist entsprechend weniger Metall im Herzen unterzubringen. Gleichzeitig kann das Einführinstrument, in der Regel ein Katheter, schlanker ausgeführt werden.

Vor allem bei Kombination einzelner oder mehrerer der vorbeschriebenen Merkmale und Maßnahmen ergibt sich ein Verschlusselement für ungewollte Öffnungen im Herzen mit zwei beabstandeten jeweils aus Fäden oder einem Geflecht gebildeten Verschlussscheiben zwischen welchen ein Bereich geringeren Querschnitts vorgesehen ist, der zumindest weitgehend von den beiden Fassungen oder Hülsen gebildet ist, womit die Fäden oder Geflechte der Verschlussscheiben gehalten werden, so dass an den jeweils voneinander abgewandten Außenseiten des Verschlusselements keine derartige Hülse oder Fassung notwendig ist. Ferner ist jede Verschlussscheibe einzeln herstellbar, was zu einem einfacheren Herstellungs- oder Flechtvorgang führt, als wenn beide Verschlussscheiben durch einen gemeinsamen Flechtvorgang gebildet werden.

Nachstehend ist ein Ausführungsbeispiel der Erfindung anhand der Zeichnung näher beschrieben. Es zeigt in zum Teil schematisierter Darstellung:
- Fig. 1: ein erfindungsgemäßes Verschlusselement für ungewollte Öffnungen im Herzen mit zwei beabstandeten, aus einem Geflecht gebildeten Verschlussscheiben, deren Geflechte jeweils von Fassungen gehalten sind, wobei diese Fassungen der beiden Verschlussscheiben einander zugewandt und miteinander verbunden sind und an einer dieser Fassungen ein durch das Geflecht der einen Verschlussscheibe hindurchreichendes Einführinstrument angreift,
- Fig. 2: die beiden Verschlussscheiben des erfindungsgemäßen Verschlusselements vor ihrer gegenseitigen Verbindung sowie
- Fig. 3: eine Ansicht einer Verschlussscheibe und der ihr Geflecht zusammenhaltenden hülsenartigen Fassung.

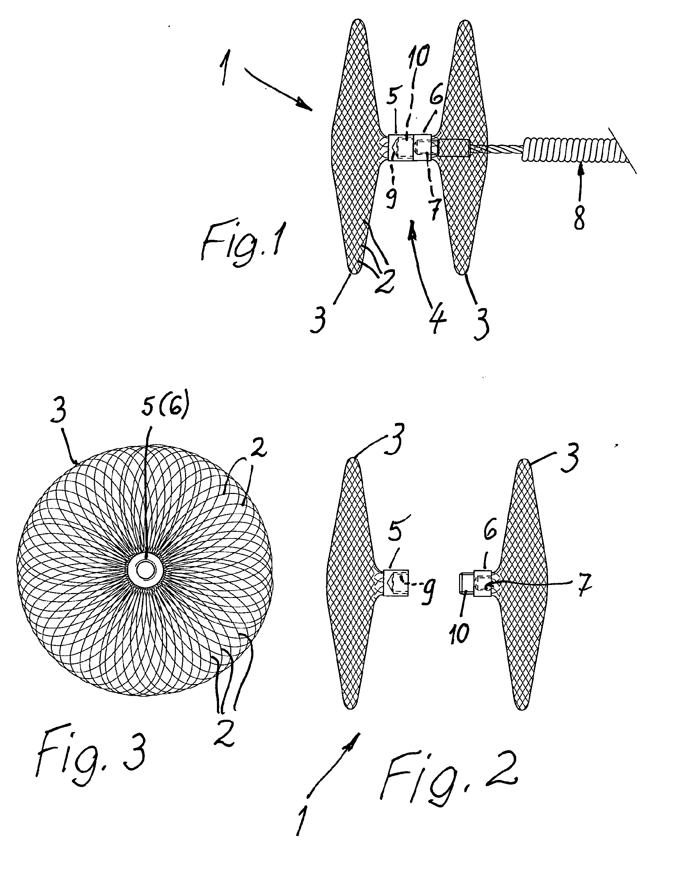

Ein im ganzen mit 1 bezeichnetes, vor allem in Fig. 1 dargestelltes Verschlusselement ist zum Einsetzen in ungewollte Öffnungen im Herzen als ASD- oder Occlusionsinstrument gedacht und vorgesehen, könnte aber auch als PDA- oder VSD-Verschluss benutzt werden, wie es beispielsweise auch aus DE 10 2006 040 415 B3 und weiteren darin genannten Druckschriften bekannt ist.

Dieses Verschlusselement 1 ist aus Memory-Materialfäden 2, beispielsweise aus Nitinolfäden oder aus mit Kunststoff ummantelten Drähten oder Fäden oder auch aus Memory-Kunststoff-fäden gebildet und vorzugsweise geflochten, könnte aber auch aus Fäden 2 aus körperverträglichem Metall und/oder Kunststoff, z.B. aus absorbierbarem Kunststoff bestehen. Vor allem bei gemeinsamer Betrachtung der Figuren 1 und 2 mit Fig. 3 wird deutlich, dass diese Fäden 2 im Ausführungsbeispiel ein Geflecht bilden.

Gemäß Fig. 1 weist das Verschlusselement 1 zwei aus dem Geflecht gebildete Verschlussscheiben 3 und zwischen diesen einen die Öffnung im Herzen in Gebrauchsstellung durchsetzenden Bereich 4 geringerer radialer Abmessung auf, wobei die Verschlussscheiben 3 in Gebrauchsstellung die Ränder der ungewollten Öffnung im Herzen beidseits übergreifen und der Bereich 4 die Öffnung durchsetzt. Dabei liegen in Gebrauchsstellung die Verschlusscheiben 3 beidseits der die Öffnung aufweisenden Wandung des Herzens an dieser an und sorgen in bekannter Weise dafür, dass die ungewollte Öffnung verschlossen wird.

Anhand der Fig. 2 wird deutlich, dass beide Verschlussscheiben 3 jeweils eine die sie bildenden Fäden 2 an einer Seite festlegende, von der Verschlussscheibe 3 in radialer Richtung überragte Fassung 5 oder 6 aufweisen und dass diese Fassungen 5 und 6 zur Bildung eines Abstandes zwischen den beiden Verschlussscheiben 3 gemäß Fig. 1 und zur Bildung des Bereichs 4 mit der verminderten radialen Abmessung in Gebrauchsstellung miteinander in noch zu beschreibender Weise verbunden sind. Somit ergibt sich ein Verschlusselement 1, bei welchem die Fassungen 5 und 6 für die Geflechte der Verschlussscheiben 3 nicht an deren Außenseiten, sondern in Gebrauchsstellung zwischen diesen und damit innerhalb der ungewollten Öffnung im Herzen angeordnet sind, also den Blutstrom in den Herzvorhöfen praktisch nicht beeinträchtigen können.

In den Fig. 1 und 2 erkennt man außerdem, dass die Fassung 6 einer der Verschlussscheiben 3 eine von der noch zu erläuternden Verbindungsstelle mit der Fassung 5 der anderen Verschlussscheibe 3 abgewandte Kupplungsstelle oder Öffnung 7 zum Verbinden mit einem in Fig. 1 angedeuteten Zuführinstrument 8 aufweist, wobei die Maschenweite des Geflechts der Verschlussscheibe 3 dem Durchtritt dieses Zuführinstruments 8 freigibt oder freilässt, zumal die Fäden 2 des Geflechts genügend biegsam und nachgiebig sind, um das distale Ende des Zuführinstruments 8 in die Kupplungsstelle oder Öffnung 7 einführen zu können.

Gemäß Fig. 1 ergibt sich also, dass die in Gebrauchsstellung einander zugewandten Fassungen 5 und 6 der beiden Verschlussscheiben 3 zugfest miteinander verbunden sind, um mit Hilfe des Zuführinstruments 8 implantiert und platziert werden zu können und dann in Gebrauchsstellung die ungewollte Öffnung im Herzen sicher und ohne die Gefahr einer gegenseitigen Trennung verschließen können.

Gemäß Fig. 2 ist vorgesehen, dass die Fassung 5 der einen Verschlussscheibe 3 eine axiale, nach außen reichende Öffnung oder Ausnehmung 9 hat, in welcher ein Vorsprung 10 der Fassung 6 der zweiten Verschlussscheibe 3 einführbar und in dieser Lage damit verbindbar ist, wobei Fig. 2 die noch getrennte und Fig. 1 die zusammengefügte Position darstellt.

Der Vorsprung 10 der Fassung 6 passt dabei formschlüssig in die Öffnung oder Ausnehmung oder Vertiefung 9 der anderen Fassung 5 und kann damit verschraubt und/oder verschweißt sein, wenn die gegenseitige Verbindung vollendet ist.

Denkbar ist aber auch, dass die axial verlaufende Öffnung, oder Ausnehmung oder Vertiefung 9 der Fassung 5 der einen Verschlussscheibe 3 ein Innengewinde und der dazu passende Vorsprung 10 der Fassung 6 der anderen Verschlussscheibe 3 ein dazu passendes Gewindestück oder Außengewinde für eine gegenseitige Verschraubung der beiden Fassungen 5 und 6 aufweist, was als gegenseitige Befestigung ausreichen kann, gegebenenfalls aber zusätzlich durch eine Verschweißung gesichert werden könnte.

Die Fassung 6 weist die schon erwähnte Kupplungsstelle oder Öffnung 7 an der von der Verbindungsstelle mit der anderen Fassung 3 abgewandten Seite auf und sieht im Ausführungsbeispiel ein Gewinde vor, zu dem ein Gegengewinde am distalen Ende des Einführinstruments 8 passt, wobei zweckmäßigerweise die Kupplungsstelle oder Öffnung 7 ein Innengewinde und das Einführinstrument 8 ein dazu passendes Außengewinde haben. Somit kann das Einführinstrument 8 zug- und druckfest mit dem Verschlusselement 1 in der in Fig. 1 dargestellten Weise gekuppelt werden, um das Verschlusselement 1 in seine Gebrauchsstellung zu bringen, wonach das Einführinstrument 8 durch eine gegenläufige Drehung wieder losgeschraubt und entfernt werden kann.

Die Kupplungsstelle oder Öffnung 7 für das Einführinstrument 8 ist dabei an derjenigen Fassung vorgesehen, welche den Vorsprung 10 zum Kuppeln mit der Fassung 5 und deren Öffnung oder Ausnehmung 9 der anderen Verschlussscheibe 3 trägt, weil an der Fassung 5 durch die erwähnte Öffnung oder Ausnehmung 9 eine zusätzliche Kupplungsstelle oder Öffnung 7 nicht zulassen würde, ohne die Fassung 5 zu verlängern.

Die Außenkontur der Verschlussscheiben 3 ist im Ausführungsbeispiel gemäß Fig. 3 kreisrund, könnte aber auch von einem Kreis abweichend geformt sein und entgegen dem Ausführungsbeispiel, bei welchem beide Verschlussscheiben 3 gleich groß sind, könnten diese auch unterschiedliche Größen haben.

Es sei noch erwähnt, dass anstelle der unmittelbaren Verbindung der beiden Fassungen 5 und 6, wie sie im Ausführungsbeispiel vorgesehen ist, auch eine Verbindung denkbar wäre, bei der noch ein Zwischenstück vorgesehen ist, falls die Abmessungen einerseits der Fassungen 5 und 6 und andererseits einer zu verschließenden Öffnung im Herzen dies sinnvoll oder notwendig erscheinen lässt.

Das Verschlusselement 1 dient zum Verschließen einer ungewollten Öffnung im Herzen und weist dazu zwei aus einem Geflecht oder Fäden 2 gebildete Verschlussscheiben 3 auf, wobei die Fäden 2 und die Geflechte dieser Verschlussscheiben durch eine Hülse oder Fassung 5 und 6 gehalten sind. Die beiden Verschlussscheiben 3 sind dabei zunächst getrennt und können zur Bildung der endgültigen Form des Verschlusselements 1 mit ihren beiden Fassungen 5 und 6 zueinander weisend verbunden werden, so dass die Fassungen 5 und 6 einen Abstand zwischen den beiden Verschlussscheiben 3 und einen Bereich 4 mit einer gegenüber den parallel angeordneten Verschlussscheiben 3 verminderten radialen Abmessung bilden, so dass an den voneinander abgewandten Außenseiten oder Stirnseiten des Verschlusselements 1 und seiner Verschlussscheiben 3 keine derartige Fassung für die Geflechte oder Fäden vorstehen.

## Patentansprüche

1. Verschlusselement (1) für ungewollte Öffnungen im Herzen oder für Vorhofseptum-Defekte (ASD), welche aus Fäden (2) aus körperverträglichem Metall und/oder Kunststoff, aus absorbierbarem Kunststoff, aus Memory-Material-Fäden (2), aus Memory-Kunststoff-Fäden, aus Nitinoldrähten oder aus mit Kunststoff ummantelnden Drähten oder Fäden gebildet oder geflochten ist, wobei dieses Verschlusselement (1) zwei aus den Fäden (2) gebildete Verschlussscheiben (3) und zwischen diesen einen die Öffnung im Herzen in Gebrauchsstellung durchsetzenden Bereich (4) geringerer radialer Abmessung aufweist und die Verschlussscheiben (3) die Ränder der ungewollten Öffnung im Herzen in Gebrauchsstellung beidseits übergreifen, wobei beide Verschlussscheiben (3) jeweils eine die sie bildenden Fäden (2) an einer Seite festlegende, von der Verschlussscheibe (3) in radialer Richtung überragte Fassung (5, 6) aufweisen, diese Fassungen (5, 6) zur Bildung eines Abstands zwischen den beiden Verschlussteilen (3) und zur Bildung des Bereichs (4) mit der verminderten radialen Abmessung in Gebrauchsstellung miteinander verbunden sind, die Fassung (5) der einen Verschlussscheibe (3) eine axiale, nach außen reichende Öffnung oder Ausnehmung (9) hat, in welche ein Vorsprung (10) der Fassung (6) der zweiten Verschlussscheibe (3) einführbar und in dieser Lage damit verbindbar ist und wobei der Vorsprung (10) der einen Fassung (6) in die Öffnung oder Ausnehmung oder Vertiefung (9) der anderen Fassung (5)formschlüssig passt, damit verschraubbar und/oder verschweißbar ist, **dadurch gekennzeichnet, dass** eine einzige Fassung (6) der beiden Fassungen (5,6) eine von der Verbindungsstelle mit der anderen Fassung (5) abgewandte Kupplungsstelle oder Öffnung (7) für den lösbaren Angriff eines Einführinstruments (8) aufweist, dass die Kupplungsstelle oder Öffnung (7) ein Gewinde hat, zu dem ein Gegengewinde des Einführinstruments (8) passt, wobei die Fäden (2) oder die Maschenweite des Geflechts der Verschlussscheibe (3) einen Durchtritt für das Zuführinstrument (8) freigibt oder frei lässt, und dass die Kupplungsstelle oder Öffnung (7) für das Einführinstrument (8) an derjenigen Fassung (6) vorgesehen ist, welche den Vorsprung (10) zum Kuppeln mit der Fassung (5) der anderen Verschlussscheibe (3) trägt.

2. Verschlusselement nach Anspruch 1, **dadurch gekennzeichnet, dass** die in Gebrauchsstellung einander zugewandten Fassungen (5, 6) der beiden Scheiben (3) zugfest miteinander verbunden sind.

3. Verschlusselement nach einem Anspruch 1 oder 2, **dadurch gekennzeichnet, dass** die axial verlaufende Öffnung oder Ausnehmung (9) der Fassung (5) der einen Verschlussscheibe (3) ein Innengewinde und der dazu passende Vorsprung (10) der Fassung (6) der zweiten Verschlussscheibe (3) ein dazu passendes Gewindestück oder Außengewinde für eine gegenseitige Verschraubung der beiden Fassungen (5, 6) aufweist.

## Claims

1. Occlusion element (1) for unwanted openings in the heart or for atrial septal defects (ASDs), which is formed or braided from threads (2) of biocompatible metal and/or plastic, from absorbable plastic, from memory-material threads (2), from memory-plastic threads, from nitinol wires or from wires or threads covered with plastic, wherein this occlusion element (1) comprises two occlusion discs (3) formed from the threads (2), and, between these discs, a region (4) of smaller radial dimension which passes through the opening in the heart in the usage position, and the occlusion discs (3) engage over the edges of the unwanted opening in the heart on both sides in the usage position, wherein the two occlusion discs (3) comprise a respective holder (5, 6), which fixes the threads (2) forming them on one side and beyond which the occlusion disc (3) protrudes in the radial direction, these holders (5, 6) are connected to one another in the usage position to form a spacing between the two occlusion parts (3) and to form the region (4) with the reduced radial dimension, the holder (5) of one occlusion disc (3) has an axial outwardly extending opening or recess (9) into which a projection (10) of the holder (6) of the second occlusion disc (3) can be inserted and can be connected thereto in this position, and wherein the projection (10) of one holder (6) fits in a form-fit manner into the opening or recess or depression (9) of the other holder (5), can be screwed and/or welded thereto, **characterised in that** one holder (6) of the two holders (5, 6) comprises a coupling point or opening (7), facing away from the point of connection to the other holder (5), for releasable engagement of an insertion instrument (8), that the coupling point or opening (7) has a thread with which a counter thread on the insertion instrument (8) fits, wherein the threads (2) or mesh width of the braiding of the occlusion disc (3) opens or leaves free a passage for the delivery instrument (8), and that the coupling point or opening (7) for the insertion instrument (8) is provided on the holder (6) which bears the projection (10) for coupling to the holder (5) of the other occlusion disc (3).

2. Occlusion element as claimed in claim 1, **characterised in that** the holders (5, 6), which face one another in the usage position, of the two discs (3) are connected to one another in a tension-proof manner.

3. Occlusion element as claimed in any one of claims 1 or 2, **characterised in that** the axially extending opening or recess (9) of the holder (5) of one occlusion disc (3) has an internal thread and the projection (10), which fits together therewith, of the holder (6) of the second occlusion disc (3) has a threaded piece or external thread fitting together therewith for mutual screw-fastening of the two holders (5, 6).

## Revendications

1. Élément de fermeture (1) pour des ouvertures non souhaitées dans le coeur ou pour des défauts du septum interauriculaire (ASD), qui est formé ou tressé à partir de fils (2) en métal et/ou matière plastique compatible avec le corps, en matière plastique absorbable, en fils de matériau à mémoire (2), en fils de matière plastique à mémoire, en fils de nitinol ou en fils ou filaments enrobés de matière plastique, dans lequel cet élément de fermeture (1) présente deux disques de fermeture (3) formés des fils (2) et entre ceux-ci une zone (4) de dimension radiale plus faible traversant l'ouverture dans le coeur en position d'utilisation et les disques de fermeture (3) recouvrent de part et d'autre en position d'utilisation les bords de l'ouverture non souhaitée dans le coeur, dans lequel les deux disques de fermeture (3) présentent respectivement une monture (5, 6) fixant les fils (2) qui les forment sur un côté et saillante sur le disque de fermeture (3) en direction radiale, ces montures (5, 6) sont reliées l'une à l'autre en position d'utilisation pour la formation d'une distance entre les deux disques de fermeture (3) et pour la formation de la zone (4) de moindre dimension radiale, la monture (5) d'un premier disque de fermeture (3) comporte une ouverture ou une découpe axiale (9) dirigée vers l'extérieur, dans laquelle une saillie (10) de la monture (6) du deuxième disque de fermeture (3) peut être insérée et ainsi assemblée dans cette position, et dans lequel la saillie (10) d'une première monture (6) s'ajuste par emboîtement dans l'ouverture ou la découpe ou le creux (9) de l'autre monture (5) et peut être vissée et/ou soudée à celle-ci, **caractérisé en ce qu'**une seule monture (6) des deux montures (5, 6) présente une zone de couplage ou une ouverture (7) située à l'opposé de la zone d'assemblage avec l'autre monture pour l'application amovible d'un instrument d'introduction (8), **en ce que** la zone de couplage ou l'ouverture (7) présente un filet, sur lequel s'ajuste un filet opposé de l'instrument d'introduction (8), dans lequel les fils (2) ou la largeur des mailles du tressage du disque de fermeture (3) dégage ou libère un passage pour l'instrument d'introduction (8) et **en ce que** la zone de couplage ou l'ouverture (7) pour l'instrument d'introduction (8) est prévue sur la monture (6) qui porte la saillie (10) à coupler avec la monture (5) de l'autre disque de fermeture (3).

2. Élément de fermeture selon la revendication 1, **caractérisé en ce que** les montures (5, 6) des deux disques (3) tournées l'une vers l'autre en position d'utilisation sont assemblées l'une à l'autre de façon à résister à la traction.

3. Élément de fermeture selon l'une quelconque des revendications 1 ou 2, **caractérisé en ce que** l'ouverture ou la découpe (9) orientée axialement de la monture (5) du premier disque de fermeture (3) présente un filet intérieur et la saillie (10) adaptée à celle-ci de la monture (6) du deuxième disque de fermeture (3) présente une pièce filetée ou un filet extérieur correspondant pour un assemblage vissé mutuel des deux montures (5, 6).
